# EUROPÄISCHE PATENTANMELDUNG

(11) **EP 2 468 190 A1**
(43) Veröffentlichungstag der Anmeldung: **27.06.2012**
(21) Anmeldenummer: 10016067.0
(22) Anmeldetag: 23.12.2010
(51) Int. Cl.: A61B 8/00, G01S 15/89, G10K 1/00

(54) **Ultraschall-Sonde, Ultraschall-Monitoring-System sowie deren Verwendung**

(71) Anmelder: em-tec GmbH, 86923 Finning (DE)
(72) Erfinder: Heinze, Werner, Dipl.-Ing., 86899 Landsberg (DE); Esterl, Stefan, Dr.-Ing., 82194 Gröbenzell (DE); Bober, Maciej, Dipl.-Ing., 86919 Utting (DE)
(74) Vertreter: Pfenning, Meinig & Partner GbR

(57) **Zusammenfassung**

Die Erfindung betrifft eine Ultraschall-Sonde enthaltend ein Sonden-Gehäuse, in dem mindestens ein Ultraschall-Wandler in mindestens einem Wandler-Gehäuse angeordnet ist sowie mindestens zwei Antriebselemente für das Wandler-Gehäuse, wobei das Sonden-Gehäuse zumindest bereichsweise mit einem Ultraschall übertragenden Medium gefüllt ist. Weiterhin betrifft die Erfindung ein Ultraschall-Monitoring-System, in dem eine derartige Ultraschall-Sonde zusammen mit einer Sondensteuerung, einem Ultraschall-Generator, einer Signalanzeige sowie einer Haltevorrichtung enthalten ist. Weiterhin betrifft die Erfindung die Verwendung der Ultraschall-Sonden für das Monitoring von Schlaganfall-Patienten, das Monitoring bei Operationen sowie das Monitoring von Dialyse-Shunts.

## Beschreibung

Die Erfindung betrifft eine Ultraschall-Sonde enthaltend ein Sonden-Gehäuse, in dem mindestens ein Ultraschall-Wandler in mindestens einem Wandler-Gehäuse angeordnet ist sowie mindestens zwei Antriebselemente für das Wandler-Gehäuse, wobei das Sonden-Gehäuse zumindest bereichsweise mit einem Ultraschall übertragenden Medium gefüllt ist. Weiterhin betrifft die Erfindung ein Ultraschall-Monitoring-System, in dem eine derartige Ultraschall-Sonde zusammen mit einer Sondensteuerung, einem Ultraschall-Generator, einer Signalanzeige sowie einer Haltevorrichtung enthalten ist. Weiterhin betrifft die Erfindung die Verwendung der Ultraschall-Sonden für das Monitoring von Schlaganfall-Patienten, das Monitoring bei Operationen sowie das Monitoring von Dialyse-Shunts.

In der Gefäßdiagnostik ist der Ultraschall-Doppler in der heutigen Medizin unverzichtbar, da die Anwendung einfach, nicht-invasiv sowie kontinuierlich durchführbar ist.

Das Prinzip basiert darauf, dass eine Ultraschall-Sonde über ein akustisches Kopplungsmedium mit dem zum Gefäß benachbarten Gewebe in Kontakt gebracht wird. Es erfolgt eine Beschallung des zu untersuchenden Gefäßes. Die roten Blutkörperchen wiederum reflektieren das Signal und verursachen einen Doppler-Shift. Die Sonde wird in der Regel mit einer Frequenz zwischen 2 und 8 MHz betrieben.

Seit 1982 wird der transkranielle Doppler (TCD) zur Blutflussdiagnostik der Hirngefäße eingesetzt. Dabei wird ein gepulster Doppler verwendet, d.h. die Sonde enthält nur einen Piezokristall, welcher abwechselnd als Sender und Empfänger geschaltet ist. Durch Einstellen des Zeitfensters für den Empfang lässt sich die Tiefe festlegen, aus der ein Echo ausgewertet werden soll.

Der transkranielle Doppler hat ein breit gefächertes Anwendungsfeld, insbesondere auch in länger andauernden Untersuchungen. Die Schwierigkeit besteht allerdings darin, die Gefäße in der Tiefe zu orten und das Signal durch Bewegung nicht wieder zu verlieren.

Im Stand der Technik wurden diese Probleme bislang durch den Einsatz von Fixiervorrichtungen gelöst, z.B. helmartige Fixiervorrichtungen, in die eine Doppler-Sonde integriert ist (US 5,070,880 und US 5,409,005 und US 6,547,737 B2). Derartige Systeme haben allerdings den Nachteil, dass derartige Helme das Wohlbefinden des Patienten stark einschränken können.

Aus der DE 298 23 361 U1 ist eine Ultraschall-Vorrichtung bekannt, die eine am menschlichen Kopf befestigbare Halteeinheit aufweist, an welcher die Sondeneinheit verstell- und daraufhin fixierbar ist, um bei möglicher Kopfbewegung die Ultraschallsonde stets in der gewählten, optimalen Position zu halten. Auch bei diesem System erfolgt demnach eine Änderung der Position der Ultraschall-Sonde in der Halteeinheit. Auch hier tritt demnach der Nachteil auf, dass die Positionierung nicht nur zur Halteeinheit, sondern auch zum menschlichen Kopf verändert wird und das Wohlbefinden des Patienten stark einschränkt.

Ausgehend hiervon war es Aufgabe der vorliegenden Erfindung, eine Ultraschall-Sonde bereitzustellen, die nach Einstellung einer optimalen Signalstärke dieses Signal auch bei Bewegung des zu untersuchenden Körperteils beibehalten kann.

Diese Aufgabe wird durch die gattungsgemäße Ultraschall-Sonde mit den kennzeichnenden Merkmalen des Anspruchs 1 und das Ultraschall-Monitoring-System mit den Merkmalen des Anspruchs 15 gelöst. Die weiteren abhängigen Ansprüche zeigen vorteilhafte Weiterbildungen auf. In Anspruch 17 werden erfindungsgemäße Verwendungen angegeben.

Erfindungsgemäß wird eine Ultraschall-Sonde bereitgestellt, die ein Sonden-Gehäuse aufweist, in dem mindestens ein Ultraschall-Wandler in mindestens einem Wandler-Gehäuse angeordnet ist. Weiterhin sind mindestens zwei Antriebselemente für das Wandler-Gehäuse vorgesehen. Das Sonden-Gehäuse ist dabei zumindest bereichsweise mit einem Ultraschall übertragenden Medium gefüllt.

Die erfindungsgemäße Ultraschall-Sonde zeichnet sich dadurch aus, dass das mindestens eine Wandler-Gehäuse mit den mindestens zwei Antriebselementen in mechanischer Verbindung steht und damit das mindestens eine Wandler-Gehäuse in seiner räumlichen Position relativ zum Sonden-Gehäuse ausrichtbar ist.

In einer ersten bevorzugten Ausführungsform ist dabei das mindestens eine Wandler-Gehäuse an drei Punkten mit jeweils einem Antriebselement mechanisch verbunden. Somit kann die Positionierung des Wandler-Gehäuses relativ zum Sonden-Gehäuse über drei verschiedene Antriebselemente eingestellt werden, was eine größtmögliche Flexibilität bezüglich der Ausrichtung des Wandler-Gehäuses ermöglicht.

Eine zweite erfindungsgemäße Ausführungsform sieht vor, dass das mindestens eine Wandler-Gehäuse an einem Punkt am Sonden-Gehäuse form- oder stoffschlüssig fixiert ist. An zwei anderen Punkten ist das Wandler-Gehäuse mit jeweils einem Antriebselement mechanisch verbunden. Über diese beiden Antriebselemente kann dann die Position des Wandler-Gehäuses verändert werden.

Eine weitere erfindungsgemäße Ausführungsform sieht vor, dass das mindestens eine Wandler-Gehäuse einen Stempel aufweist. Dieser Stempel befindet sich in zwei Lagerungen, wobei der Stempel mit jeweils einem Antriebselement mechanisch verbunden ist, so dass durch das Antriebselement eine Änderung der Position des Stempels und damit des Wandler-Gehäuses erfolgt. Ebenso ist es möglich, dass die Antriebselemente nicht am Stempel, sondern direkt am Wandler-Gehäuse angreifen.

Als Antriebselemente kommen bevorzugt Linearantriebe, Seilzüge und/oder Federaufhängungen in Frage.

Allen Varianten ist gemein, dass eine freie Ausrichtung des Wandler-Gehäuses in alle drei Raumrichtungen ermöglicht wird.

Der mindestens eine Ultraschall-Wandler ist vorzugsweise ein ultraschallerzeugendes Element, besonders bevorzugt ein Piezo-Kristall.

Es ist weiter bevorzugt, dass das Ultraschall-übertragende Medium ein flüssiges Medium mit einer akustischen Impedanz im Bereich von 0,6 bis 3,5 MegaRayl (1 Rayl = 1 kg/sm²) ist. Besonders bevorzugt sind hier Wasser bzw. andere organische flüssige Medien, die den Ultraschall transportieren. Hierzu zählen Wasser, Alkohole, Glycerin und/oder Öle.

Vorzugsweise weist die Ultraschall-Sonde eine obere für Ultraschall zumindest teilweise durchlässige Abdeckung zum Inkontaktbringen mit dem menschlichen Körper auf. Hierbei handelt es sich bevorzugt um einen Deckel oder eine Membran. Beim Inkontaktbringen mit dem menschlichen Körper wird dann auf die zu untersuchende Körperstelle ein akustisches Kopplungsgel aufgetragen, um den Übergang zwischen Ultraschall-Sonde und menschlichem Körper luftfrei zu ermöglichen.

Eine bevorzugte Variante sieht vor, dass im Sonden-Gehäuse der Bereich zwischen der oberen Abdeckung und dem Wandler-Gehäuse mit einem den Ultraschall übertragenden Medium gefüllt ist. Der restliche Bereich, d.h. der Bereich hinter dem Ultraschall-Wandler (betrachtet in Richtung der oberen Abdeckung), ist mit einem den Ultraschall nicht übertragenden Medium, z.B. Luft, versehen.

Diesbezüglich ist es bevorzugt, dass das Wandler-Gehäuse hinter dem Ultraschall-erzeugenden Element ein sog. Backing aufweist. Hierbei handelt es sich um einen Luftspalt, der verhindert, dass eine Abstrahlung von Ultraschallwellen nach hinten, also in die vom menschlichen Körper abgewandte Richtung, erfolgt. Ein derartiges Backing kann z.B. dadurch realisiert werden, dass auf der der oberen Abdeckung abgewandten Seite des Ultraschall-erzeugenden Elementes mindestens ein Steg angeordnet ist, auf dem eine Abdeckplatte befestigt ist, wodurch sich zwischen den Stegen Lufträume bilden können.

Eine weitere bevorzugte Ausführungsform sieht vor, dass das Wandler-Gehäuse auf der der oberen Abdeckung zugewandten Seite des Ultraschall-Wandlers eine Scheibe mit einer kegelförmigen Vertiefung zur Fokussierung des Ultraschalls aufweist.

Eine weitere Variante sieht vor, dass die Ultraschall-Sonde eine Linse aufweist, die zwischen oberer Abdeckung und Wandler-Gehäuse angeordnet ist. So kann die Linse z.B. auf dem Wandler-Gehäuse aufgeklebt sein.

Es ist weiter bevorzugt, dass die Komponenten der Ultraschall-Sonde, insbesondere das Sonden-Gehäuse, das Wandler-Gehäuse und das Ultraschall übertragende Medium, aus biokompatiblen Materialien bestehen, wodurch der problemlose Einsatz im Gesundheits-Sektor ermöglicht wird.

Erfindungsgemäß wird ebenso ein Ultraschall-Monitoring-System bereitgestellt, das die folgenden Komponenten enthält:
- eine zuvor beschriebene Ultraschall-Sonde,
- eine Sondensteuerung,
- einen Ultraschall-Generator,
- eine Signalanzeige für das Monitoring des Ultraschall-Signals sowie
- eine Haltevorrichtung zur Fixierung der Ultraschall-Sonde am menschlichen Körper.

Vorzugsweise ist die Haltevorrichtung ein Helm oder eine Brille.

Das ultraschallerzeugende Element erzeugt vorzugsweise ein Ultraschallsignal mit einer Frequenz im Bereich von 2 MHz bis 16 MHz, bevorzugt von 2 MHz bis 8 MHz.

Verwendung finden die erfindungsgemäßen Ultraschall-Sonden für das Monitoring bei Herzoperationen, Operationen an den Schlagadern sowie Operationen am Gehirn. Ebenso kann die Ultraschall-Sonde für das Monitoring von Schlaganfall-Patienten sowie für das Monitoring von Dialyse-Shunts eingesetzt werden.

Anhand der nachfolgenden Figuren soll der erfindungsgemäße Gegenstand näher erläutert werden, ohne diesen auf die hier gezeigten speziellen Ausführungsformen einschränken zu wollen.
Fig. 1 zeigt eine erste erfindungsgemäße Ausführungsform der Ultraschall-Sonde.
Fig. 2a) und 2b) zeigen eine erfindungsgemäße Ausführungsform mit jeweils drei Antriebselementen.
Fig. 3 zeigt eine erfindungsgemäße Variante der Ultraschall-Sonde mit zwei Antriebselementen.
Fig. 4 zeigt eine erfindungsgemäße Ausführungsform mit zwei Antriebselementen sowie einem Fixierelement.
Fig. 5 zeigt eine erfindungsgemäße Ausführungsform mit einem gelagerten Stempel.
Fig. 6 zeigt ein Wandler-Gehäuse der erfindungsgemäßen Ultraschall-Sonde.

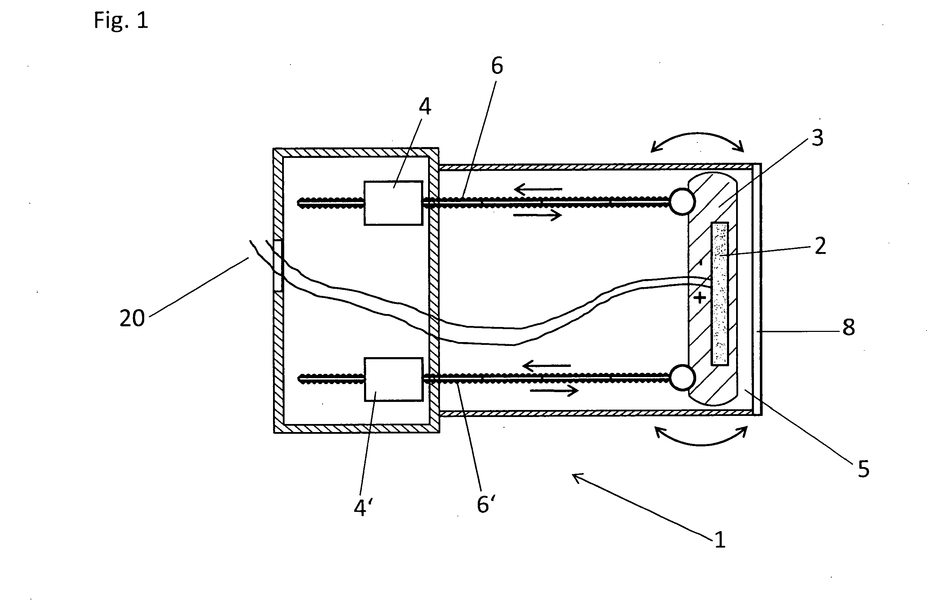

In Fig. 1 ist ein Sonden-Gehäuse 1 dargestellt, in dem der Ultraschall-Wandler 2 in einem Wandler-Gehäuse 3 untergebracht ist. Weiterhin befindet sich in dem Sonden-Gehäuse ein den Ultraschall übertragendes Medium 5. Im Sonden-Gehäuse 1 sind zusätzlich Antriebselemente 4 und 4' angeordnet, die über eine mechanische Verbindung in Form einer Stange 6 und 6' mit dem Wandler-Gehäuse 3 verbunden sind. Der Ultraschall-Wandler 2 ist über elektrische Kontaktierungen 20 mit einer Ultraschall-Basiseinheit (in der Figur nicht dargestellt) verbunden. An dem anderen Ende der Ultraschall-Sonde ist eine Abdeckung 8, z.B. in Form einer Membran, angeordnet, die als Kontaktfläche zum menschlichen Körper dient.

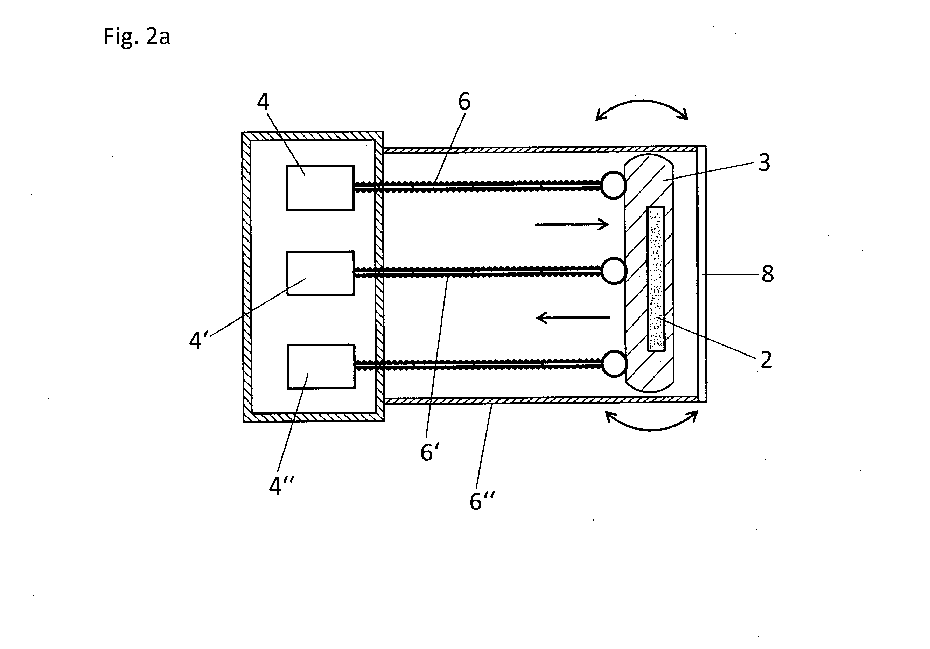

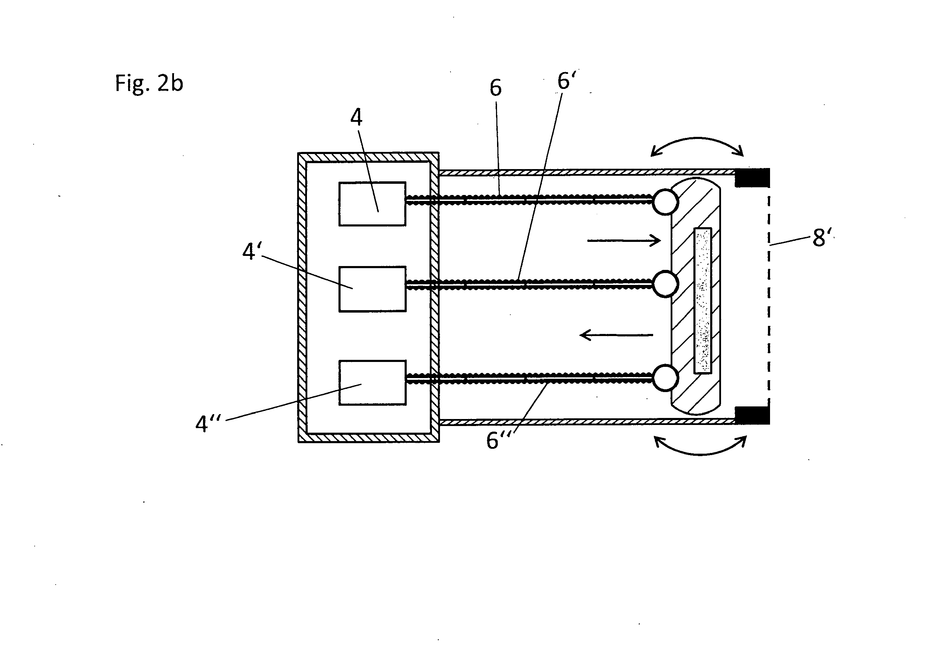

Die Figuren 2a) und 2b) sind im Wesentlichen an die Ausführungsform gemäß Figur 1 angelehnt. Allerdings sind hier drei Antriebselemente 4, 4' und 4" angeordnet, die über mechanische Verbindungen 6, 6' und 6" mit dem Wandler-Gehäuse 3, in dem ein Piezokristall 2 angeordnet ist, verbunden sind. Die Figuren 2a) und 2b) unterscheiden sich dadurch voneinander, dass die Ultraschall-Sonde gemäß Fig. 2a) eine feste Abdeckung 8 aufweist, während die Ultraschall-Sonde gemäß Fig. 2b) eine Membran 8' als Kontaktfläche aufweist.

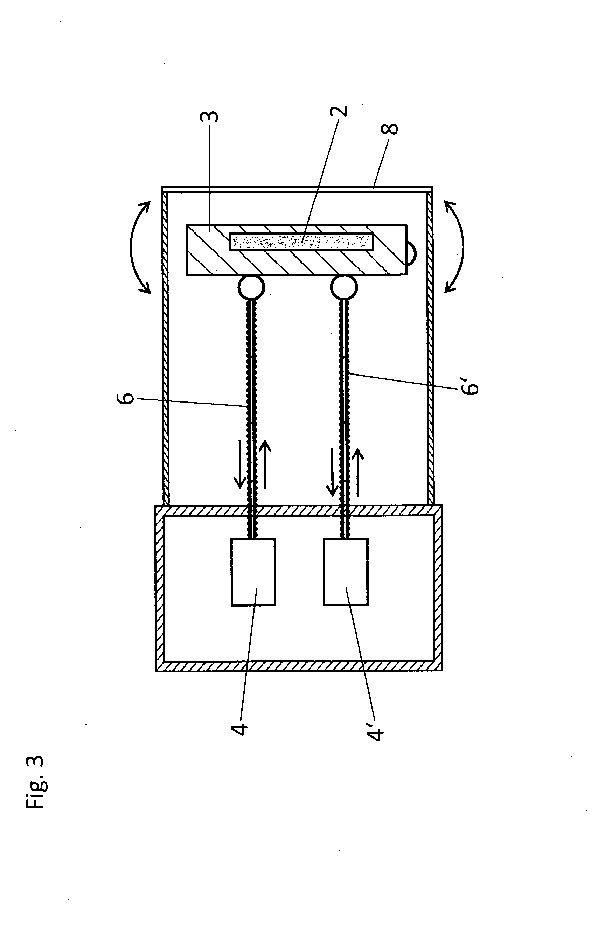

In Fig. 3 ist eine Ultraschall-Sonde dargestellt, die zwei Antriebselemente 4 und 4' aufweist, die über zwei Stangen 6 und 6' mit dem Wandler-Gehäuse 3 verbunden sind. Durch diese Anordnung ist eine Verkippung des Wandler-Gehäuses 3 möglich.

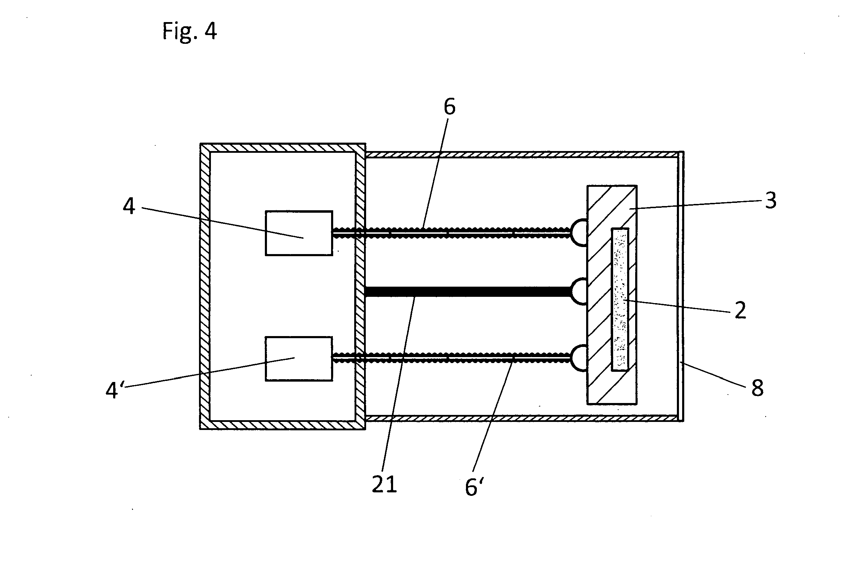

In Fig. 4 ist zusätzlich zu den beiden Antriebselementen ein Fixierelement 21 angeordnet, über das das Wandler-Gehäuse 3 fix mit dem oberen Teil des Sonden-Gehäuses 1 verbunden ist.

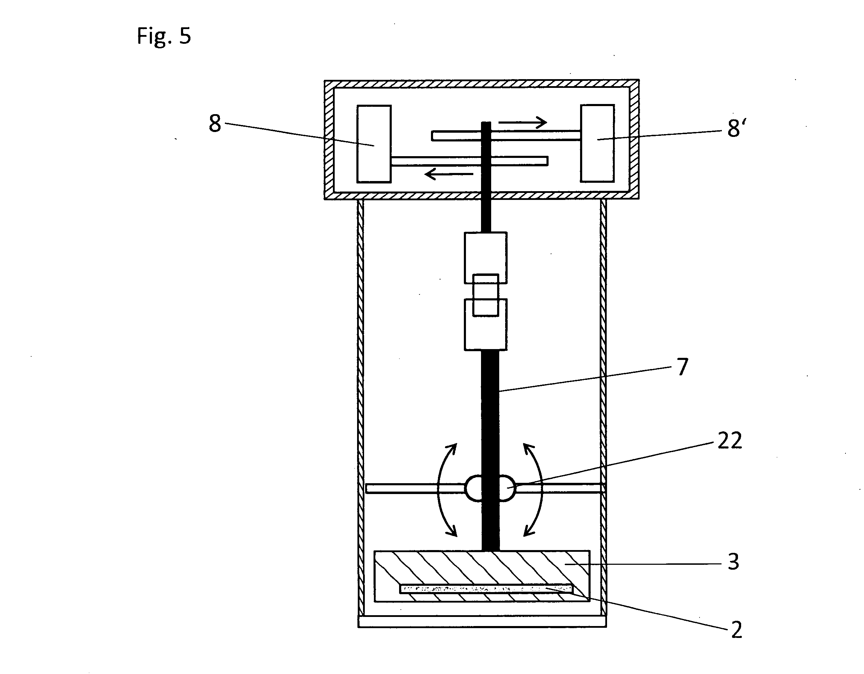

In Fig. 5 ist eine weitere Variante der Ultraschall-Sonde dargestellt, in der das Wandler-Gehäuse 3 mittels eines Stempels 7 verbunden ist. Dieser Stempel 7 durchläuft ein Lager 22 und steht am anderen Ende mit zwei Antriebselementen 8 und 8' in mechanischer Verbindung.

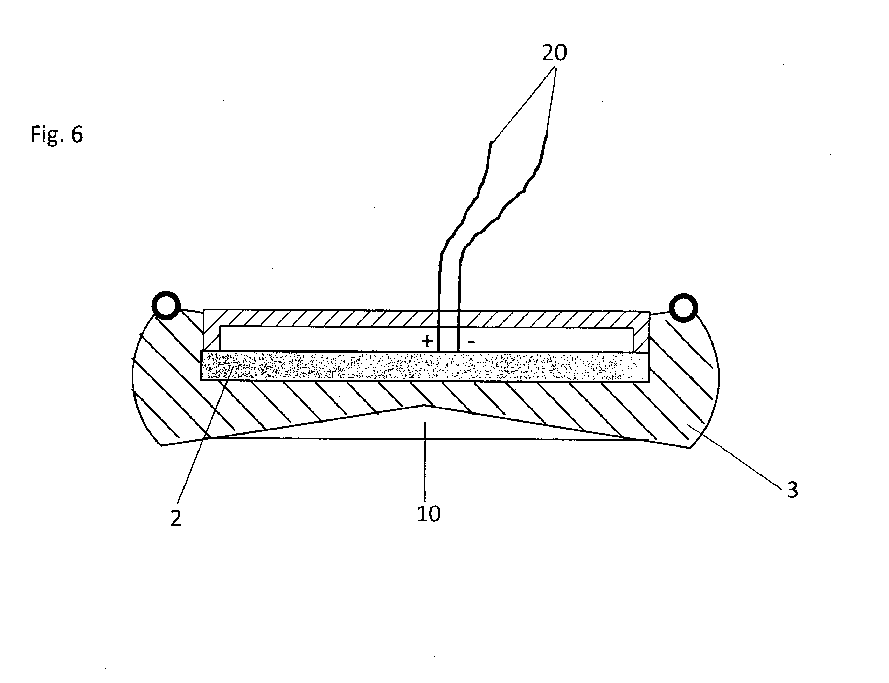

In Fig. 6 ist ein Wandler-Gehäuse 3 dargestellt, in das ein Ultraschall-Wandler 2 integriert ist. Weiterhin weist das Wandler-Gehäuse 3 auf der unteren Seite eine kegelförmige Vertiefung 10 zur Fokussierung des Ultraschalls auf.

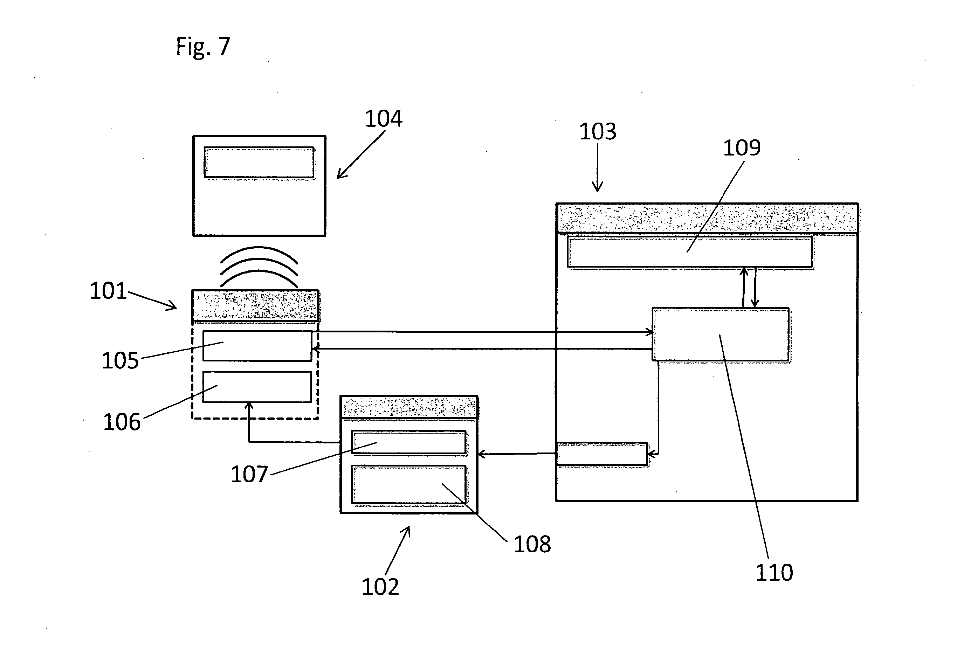

In Fig. 7 ist ein erfindungsgemäßes Ultraschall-Monitoring-System dargestellt, das aus der Ultraschall-Sonde 101, die in Wechselwirkung mit einem Patienten steht, der Steuerungseinheit 102 und der Basiseinheit 103 besteht. Die Sonde 101 weist einen Piezokristall 105 sowie einen Aktuator auf. Dabei erhält der Piezokristall 105 von der Doppler-Hardware ein Anregungssignal, während auf der anderen Seite der Piezokristall 105 ebenso Signale an die Doppler-Hardware 110 weitergibt. Dieses aufgenommene Signal veranlasst die Doppler-Hardware 110 die Steuerungseinheit 102 anzusteuern, die einen Suchalgorhythmus 107 und Kalibrationsdaten für die Sonde 108 aufweist. Anhand dieser Daten kann eine Kontrolle des Aktuators 106 erfolgen. In der Basiseinheit ist weiterhin eine Software installiert, mit der eine Optimierung des Sendesignals erfolgt.

## Patentansprüche

1. Ultraschall-Sonde enthaltend ein Sonden-Gehäuse (1), in dem mindestens ein Ultraschall-Wandler (2) in mindestens einem Wandler-Gehäuse (3) angeordnet ist, sowie mindestens zwei Antriebselemente (4, 4', 4") für das Wandler-Gehäuse (3), wobei das Sonden-Gehäuse (1) zumindest bereichsweise mit einem Ultraschall übertragenden Medium (5) gefüllt ist,
**dadurch gekennzeichnet, dass** das mindestens eine Wandler-Gehäuse (3) mit den mindestens zwei Antriebselementen (4, 4', 4") in mechanischer Verbindung (6, 6') steht und damit das mindestens eine Wandler-Gehäuse (3) in seiner räumlichen Position relativ zum Sonden-Gehäuse (1) ausrichtbar ist.

2. Ultraschall-Sonde nach Anspruch 1,
**dadurch gekennzeichnet, dass** das mindestens eine Wandler-Gehäuse (3) an drei Punkten mit jeweils einem Antriebselement (4, 4', 4") mechanisch verbunden ist.

3. Ultraschall-Sonde nach Anspruch 1,
**dadurch gekennzeichnet, dass** das mindestens eine Wandler-Gehäuse (3) an einem Punkt am Sonden-Gehäuse (1) fixiert ist und an zwei Punkten mit jeweils einem Antriebselement (4, 4') mechanisch verbunden ist.

4. Ultraschall-Sonde nach Anspruch 1,
**dadurch gekennzeichnet, dass** das mindestens eine Wandler-Gehäuse (3) einen gelagerten Stempel (7) aufweist, wobei der Stempel (7) an zwei Punkten mit jeweils einem Antriebselement (8, 8') mechanisch verbunden ist.

5. Ultraschall-Sonde nach einem der vorhergehenden Ansprüche,
**dadurch gekennzeichnet, dass** die Antriebselemente (4, 4', 4", 8, 8') Linearantriebe, Seilzüge und/oder Federaufhängungen sind.

6. Ultraschall-Sonde nach einem der vorhergehenden Ansprüche,
**dadurch gekennzeichnet, dass** das Wandler-Gehäuse (3) in drei Raumrichtungen frei ausrichtbar ist.

7. Ultraschall-Sonde nach einem der vorhergehenden Ansprüche,
**dadurch gekennzeichnet, dass** das der mindestens eine Ultraschall-Wandler (2) ein ultraschallerzeugendes Element, insbesondere ein Piezo-Kristall ist.

8. Ultraschall-Sonde nach dem vorhergehenden Anspruch,
**dadurch gekennzeichnet, dass** das ultraschallerzeugende Element ein Ultraschallsignal mit einer Frequenz im Bereich von 2 MHz bis 16 MHz, insbesondere von 2 MHz bis 8 MHz erzeugt.

9. Ultraschall-Sonde nach einem der vorhergehenden Ansprüche,
**dadurch gekennzeichnet, dass** das Ultraschall übertragende Medium (5) ein flüssiges Medium mit einer akustischen Impedanz im Bereich von 0,6 bis 3,5 MegaRayl ist, insbesondere Wasser oder organische flüssige Medien, hier insbesondere Alkohole, Glycerin oder Öle.

10. Ultraschall-Sonde nach einem der vorhergehenden Ansprüche,
**dadurch gekennzeichnet, dass** die Ultraschall-Sonde eine obere für Ultraschall zumindest teilweise durchlässige Abdeckung (9) zum Inkontaktbringen mit dem menschlichen Körper aufweist, insbesondere ein Deckel oder eine Membran.

11. Ultraschall-Sonde nach einem der vorhergehenden Ansprüche,
**dadurch gekennzeichnet, dass** im Sonden-Gehäuse (1) der Bereich zwischen der oberen Abdeckung (9) und dem Wandler-Gehäuse (3) mit einem den Ultraschall übertragenden Medium (5) und der andere Bereich mit einem den Ultraschall nicht übertragenden Medium gefüllt.

12. Ultraschall-Sonde nach einem der vorhergehenden Ansprüche,
**dadurch gekennzeichnet, dass** das Wandler-Gehäuse (3) auf der auf der der oberen Abdeckung (9) zugewandten Seite des Ultraschall-Wandlers (2) eine Scheibe mit einer kegelförmigen Vertiefung (10) zur Fokussierung des Ultraschalls aufweist.

13. Ultraschall-Sonde nach einem der vorhergehenden Ansprüche,
**dadurch gekennzeichnet, dass** die Ultraschall-Sonde eine Linse (11) aufweist, die zwischen oberer Abdeckung (9) und Wandler-Gehäuse (3) angeordnet ist.

14. Ultraschall-Sonde nach einem der vorhergehenden Ansprüche,
**dadurch gekennzeichnet, dass** die Komponenten der Ultraschall-Sonde, insbesondere das Sonden-Gehäuse (1), das Wandler-Gehäuse (3) und das Ultraschall übertragende Medium (5) aus biokompatiblen Materialien bestehen.

15. Ultraschall-Monitoring-System enthaltend
• eine Ultraschall-Sonde nach einem der vorhergehenden Ansprüche,
• eine Sondensteuerung (12),
• einen Ultraschall-Generator (13),
• eine Signalanzeige (14) für das Monitoring des Ultraschall-Signals sowie
• eine Haltevorrichtung zur Fixierung der Ultraschall-Sonde am menschlichen Körper.

16. Ultraschall-Monitoring-System nach Anspruch 14, **dadurch gekennzeichnet, dass** die Haltevorrichtung ein Helm oder eine Brille ist.

17. Verwendung der Ultraschall-Sonde nach einem der Ansprüche 1 bis 13 für das Monitoring bei Herzoperationen, Operationen an den Schlagadern sowie Operationen am Gehirn, für das Monitoring von Schlaganfall-Patienten sowie für Monitoring von Dialyse-Shunts.
